# EUROPEAN PATENT APPLICATION

(11) **EP 4 112 634 A1**
(43) Date of publication of application: **04.01.2023**
(21) Application number: 20920914.7
(22) Date of filing: 20.11.2020
(51) Int. Cl.: C07K 14/165, G01N 33/68, G01N 33/569

(54) **SARS-COV-2 RECOMBINANT N PROTEIN, AND PREPARATION METHOD AND PURIFICATION METHOD THEREFOR**

(30) Priority: 28.02.2020 CN 202010126734
(71) Applicant: Shenzhen Yhlo Biotech Co., Ltd., 518116 Shenzhen (CN)
(72) Inventor: LIN, Xiaotao, Shenzhen, Guangdong 518116 (CN); LUO, Chunlei, Shenzhen, Guangdong 518116 (CN); ZHANG, Sai, Shenzhen, Guangdong 518116 (CN); QIAN, Chungen, Shenzhen, Guangdong 518116 (CN); HU, Kunhui, Shenzhen, Guangdong 518116 (CN)
(74) Representative: Leonard, Thomas Charles
(86) International application number: PCT/CN2020/130435
(87) International publication number: WO 2021/169433

(57) **Abstract**

Provided are a recombinant N protein of SARS-CoV-2 and methods for preparing the same and for purifying the same. The recombinant N protein of SARS-CoV-2 has an N protein sequence, wherein each of both ends of the N protein sequence are linked to an oligolysine fragment. An expression method for the recombinant N protein includes: a. providing a sample including the recombinant N protein; and b. performing cation exchange chromatography resin purification treatment at least once, and collecting breakthrough substance. The method may effectively improve the expression purity of the recombinant N protein.

## Description

### TECHNICAL FIELD

The present disclosure relates to the technical field of protein purification, in particular to a recombinant N protein of SARS-CoV-2 and methods for preparing the same and for purifying the same.

### BACKGROUND

Severe Acute Respiratory Syndrome Coronavirus 2 (SARS-CoV-2) is a subtype of Severe Acute Respiratory Syndrome-related coronavirus species belonging to genus *Betacoronavirus,* family *Coronaviridae.* SARS-CoV-2 is enveloped and contains positivesense single-stranded RNA. SARS-CoV-2 caused the coronavirus disease 2019 (COVID-19) outbreak in late 2019. Its gene sequence belongs to the same lineage as, but different clades form Severe Acute Respiratory Syndrome (SARS) virus and Middle East Respiratory Syndrome (MERS) virus. The National Health Commission of the People's Republic of China has designated the acute respiratory disease caused by infection by this virus as a Class B statutory infectious disease that is managed as Class A.

The N protein is a major structural protein of coronaviruses, and it is a basic protein. Previous studies on the structural proteins of animal coronaviruses have shown that the N protein plays an important role in viral replication and pathological responses. SUMOylation of the N protein plays a major role in promoting the formation of homologous oligomers, and the N protein also plays an important role in the regulation of host cell lysis.

In the prior art, the diagnosis of COVID-19 is mainly carried out using a nucleic acid detection kit. In the nucleic acid detection method, RNA is reverse-transcribed by a reverse transcriptase into DNA, which is then amplified through PCR under the action of DNA polymerase to enhance the signal, thereby showing the existence of viral nucleic acid. However, the novel coronavirus is an RNA virus in which RNA molecules are easily degraded during sample collection, storage and transportation, leading to false negative results in nucleic acid testing. The nucleic acid detection reaction takes about 2 hours for once and requires a fluorescence quantitative PCR instrument, which means that it takes a long time and has high requirement on equipment.

In the process of virus infection, a human body will be stressed to produce corresponding antibodies to resist the infection. Therefore, an immunodiagnostic method to determine whether or not one is infected with the virus by detecting the presence of antibodies may be used as a good supplement to the nucleic acid detection method. Antibodies produced by the human body can exist stably in the blood, has high specificity and can be fast detected. As, N protein as the main structural protein of coronavirus can stimulate the body to produce a large number of specific antibodies, but there is no purification method with specificity and high-yield for the N protein of SARS-CoV-2 in the prior art. Therefore, the detection of COVID-19-related antibodies using the recombinant N protein prepared in the prior art tends to be inaccurate, which will interfere with the detection results.

In view of this, the present disclosure is proposed.

### SUMMARY

The present disclosure relates to a recombinant N protein of SARS-CoV-2 having an N protein sequence as set forth in SEQ ID NO: 1, wherein each of both ends of the N protein sequence are linked to an oligolysine fragment.

According to another aspect of the present disclosure, the present disclosure also relates to a method for purifying an N protein of SARS-CoV-2 including:
step a) providing a sample comprising the recombinant N protein as described above; and
step b) performing cation exchange chromatography resin purification treatment at least once, and collecting breakthrough substance.

The N protein sequence as set forth in SEQ ID NO: 1 is a conserved sequence of SARS-CoV-2, which is beneficial to improve the positive rate as well as the sensitivity and specificity for detection. Linking the both ends to the oligolysine fragments functions in a variety of ways. On the one hand, oligolysine fragments are not immunogenic, and thus can effectively reduce background noise and avoid the false-positive problem; on the other hand, the N protein itself has a theoretical isoelectric point of 10.51, while adding the oligolysine fragments at the both ends makes the theoretical isoelectric point more alkaline (10.93), making the target protein more efficiently bound to a cation exchange column in the purification process of cation exchange chromatography. Also, during the preparation of proteins, for example homogenization of bacteria, a small amount of RNase is usually added, which may effectively and rapidly degrade the RNA molecules bound to the target protein. Due to the high electric point, NP protein and most of other proteins can be removed efficiently during the elution process with linear salt concentration. These factors as above may effectively improve the expression purity of the recombinant N protein. The purity of the recombinant N protein purified according to the present disclosure may be up to about 95% or more.

The present disclosure further provides the nucleic acid, vector, host cell and preparation method related to the recombinant N protein.

The present disclosure further provides a composition obtained by purification by the method as described above.

The present disclosure also relates to a reagent or kit for detecting an antibody against N protein of SARS-CoV-2, comprising the recombinant N protein as described above or the composition as described above.

### BRIEF DESCRIPTION OF THE DRAWINGS

To describe the technical solutions of the embodiments of the present disclosure or the prior art more clearly, the following briefly introduces the accompanying drawings required for describing the embodiments or the prior art. Apparently, the accompanying drawings in the following description show some embodiments of the present disclosure, and persons of ordinary skill in the art may also derive other drawings from these accompanying drawings without creative efforts.

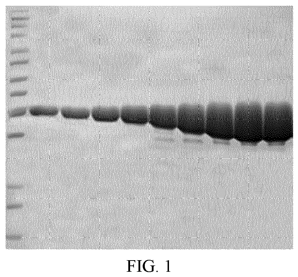

FIG. 1 shows an electrophoresis diagram of purified N protein (from 2 µg to 10 µg) at different loading amounts in one embodiment of the present disclosure.

### DETAILED DESCRIPTION OF THE EMBODIMENTS

Reference will now be provided in detail to the embodiments of the present disclosure, one or more examples of which are described below. Each example is provided by way of illustration rather than limitation to the present disclosure. In fact, it will be apparent to those skilled in the art that various modifications and variations can be made to the present disclosure without departing from the scope or spirit of the present disclosure. For example, features illustrated or described as part of one embodiment can be used in another embodiment to produce a further embodiment.

Such modifications and changes falling within the scope of the appended claims and the scope of equivalency of the claims are therefore intended to be embraced in the present disclosure. Other objects, features and aspects of the present disclosure are disclosed in or are apparent from the following detailed description. It should be understood by those of ordinary skill in the art that the present discussion is a description of exemplary embodiments only, and is not intended to limit the broader aspects of the present disclosure.

The present disclosure relates to a recombinant N protein of SARS-CoV-2 having an N protein sequence as set forth in SEQ ID NO: 1, wherein each of both ends of the N protein sequence are linked to an oligolysine fragment.

In some embodiments, the oligolysine fragments at the both ends of the N protein sequence independently comprise 4 to 6 lysines, and preferably 5 lysines.

In some embodiments, the N protein sequence is linked to the oligolysine fragment via a linker peptide.

In some embodiments, the linker peptide is a flexible linker peptide.

In some embodiments, the linker peptide has an amino acid sequence having one or more amino acids selected from the group consisting of Gly, Ser, Pro, Ala and Glu, preferably Gly and Ser.

In some embodiments, the linker peptide has 1 to 30 amino acids, and may have 1, 2, 3, 4, 5,6, 7, 8, 9, 10, 11, 12, 13, 14, 15, 16, 17, 18, 19, 20, 21, 22, 23, 24, 25, 26, 27, 28, 29 or 30 amino acids, preferably 3 to 5 amino acids, and more preferably 4 amino acids.

In some embodiments, the sequence of the linker peptide is GSGS.

The present disclosure also provides a nucleic acid capable of expressing the recombinant N protein as described above.

In some embodiments, the nucleic acid has a sequence as set forth in SEQ ID NO:2.

The present disclosure also provides a vector comprising the nucleic acid as described above.

The term "vector" refers to a nucleic acid carrier into which polynucleotides may be inserted. When a vector enables the expression of a protein encoded by a polynucleotide inserted therein, the vector is called an expression vector. The vector may be introduced into a host cell through transformation, transduction or transfection, so that the genetic material element it carries is expressed in the host cell. Vectors are well known to those skilled in the art, including but not limited to: plasmids; phagemids; cosmids; artificial chromosomes, such as yeast artificial chromosomes (YACs), bacterial artificial chromosomes (BACs) or PI derived artificial chromosomes (PACs); bacteriophages, such as λ bacteriophage or M13 bacteriophage and animal viruses, etc. The animal viruses that may be used as vectors include, but are not limited to: retroviruses (including lentiviruses), adenoviruses, adeno-associated viruses, herpes viruses (such as herpes simplex virus), poxviruses, baculoviruses, papillomaviruses, and papovaviruses (such as SV40). In some embodiments, the vector described in the present disclosure comprises regulatory elements, such as enhancers, promoters, internal ribosome entry sites (IRES), and other expression control elements (such as transcription termination signals, or polyadenylation signal and poly (U) sequence, etc.) commonly used in genetic engineering. In a specific embodiment, the vector is pET-28a(+).

The present disclosure also provides a host cell incorporating the nucleic acid as described above in genome thereof.

The term "host cell" refers to cells into which a vector can be introduced, including, but not limited to, prokaryotic cells, such as *Escherichia coli* (*E.coli*) or *Bacterium subtilis,* etc.; insect cells, such as S2 drosophila cells or Sf9, etc; or animal cells, such as fibroblasts, CHO cells, COS cells, NSO cells, HeLa cells, BHK cells, HEK 293 cells or human cells, etc. The host cell is preferably a prokaryotic cell, more preferably *E*. *coli.*

The present disclosure also relates to a method for preparing an N protein of SARS-CoV-2 including:

culturing the host cell as described above under a suitable condition, and collecting a lysate of the host cell and/or culture solution.

According to another aspect of the present disclosure, the present disclosure also relates to a method for purifying the N protein of SARS-CoV-2 including:
step a) providing a sample comprising the recombinant N protein as described above; and
step b) performing cation exchange chromatography resin purification treatment at least once, and collecting breakthrough substance.

In the present disclosure, the term "treatment" may describe a process in which a sample flows through or passes through a chromatography column, resin, membrane, filter, or other mechanisms, and shall include the continuous flow through each mechanism and the flow paused or stopped in each mechanism.

In one embodiment of the disclosure, a sample containing the recombinant N protein is provided. Any sample containing the recombinant N protein may be used in the present disclosure. A sample comprising the recombinant N protein may include, for example, cell culture (especially cell culture supernatant). As an example, the recombinant N protein may be expressed in *E. coli.*

Any cation exchange chromatography systems available to those skilled in the art that satisfy the purification conditions of recombinant N protein of SARS-CoV-2 may be used in this embodiment, for example, the matrix used for cation exchange chromatography may contain sulfonic groups, quaternary amine groups, and may also contain imidazolyl, pyridyl, quinolinyl, piperidinyl and other groups, the preferred group is sulfopropyl (SP); the preferred cation exchange chromatography resin is SP Sepharose FF; the cation exchange chromatography resin may have a pore size selected from 500 A to 100 A and a particle size selected from 15 µ m, 30 µm, 60 µm, 80 µm, etc.

The salt of the mobile phase in the cation exchange chromatography separation may be hydrogen phosphate (especially Na₂HPO₄) and NaCl, etc.

In some embodiments, the cation exchange column is first equilibrated with a buffer of about 30 mM to 70 mM Na₂HPO₄, pH 7.1-7.7 and unbound protein is washed away.

In some embodiments, the target protein is first eluted with a linear gradient of about 30 mM to 70 mM Na₂HPO₄, 0 mM to 600 mM NaCl, pH 7.1 to 7.7.

In some embodiments, NaCl in the equilibration and loading buffer used for the hydrophobic chromatographic separation is at a concentration of 500 mM to 2000 mM, for example, 500mM, 600mM, 700mM, 800mM, 900mM, 1000mM, 1100mM, 1200mM, 1300mM, 1400mM, 1500mM, 1600mM, 1700mM, 1800mM, 1900mM, 2000mM, or within a range between any two values.

Commonly used buffers for mobile phase in hydrophobic chromatography separation are PB/PBS, Tris-HCl, HEPES, acetate buffer, etc. In some embodiments, PB/PBS in the equilibration and loading buffer used for the hydrophobic chromatographic separation is at a concentration of > 20 mM, e.g., 30 mM, 40 mM, 50 mM, and the buffer is at a pH of 7.0∼9.0.

PB: Phosphate Buffer; PBS:Phosphate Buffered Saline.

In some embodiments, the breakthrough substance in step b) is a target protein fraction with a purity of 90%, and step b) further includes subjecting the breakthrough substance to dialysis treatment at least once.

In some embodiments, the method further includes, after step b), performing anion exchange chromatography resin purification treatment on the obtained product at least once, and collecting breakthrough substance.

In some embodiments, the present disclosure includes one or more steps of clarification (which is also called collecting sediment/supernatant), and the method for clarification may be carried out by one or more steps of processes such as centrifugation, microfiltration, ultrafiltration or depth filtration; the clarification may be performed, for example, after anyone of the above-mentioned steps has been completed, for example, before and after the collection of the breakthrough substance.

The present disclosure further provides a composition obtained by purification by the method as described above.

The purity of the recombinant N protein in the composition is about 80% to 97%, preferably not less than 95%.

According to yet another aspect of the present disclosure, the present disclosure also relates to a reagent or kit for detecting an antibody against N protein of SARS-CoV-2, comprising the recombinant N protein as described above or the composition as described above.

The antibody against N protein of SARS-CoV-2 is preferably derived from humans, and may also be derived from other potential hosts containing SARS-CoV-2, such as bats, pangolins, paguma larvatas, or other mammals.

The type of the antibody may be selected from IgM, IgE, IgA, IgD, or IgG.

The embodiments of the present disclosure will be described in detail below in combination with examples.

### Example

This example provides methods for preparing and for purifying a recombinant N protein of SARS-CoV-2.

The following substances were selected: a nucleocapsid protein (N protein) having the following amino acid sequence: 
MSDNGPQNQRNAPRITFGGPSDSTGSNQNGERSGARSKQRRPQGLPNNT
ASWFTALTQHGKEDLKFPRGQGVPINTNSSPDDQIGYYRRATRRIRGGDGKMKD
LSPRWYFYYLGTGPEAGLPYGANKDGIIWVATEGALNTPKDHIGTRNPANNAAIV
LQLPQGTTLPKGFYAEGSRGGSQASSRSSSRSRNSSRNSTPGSSRGTSPARMAGN
GGDAALALLLLDRLNQLESKMSGKGQQQQGQTVTKKSAAEASKKPRQKRTATK
AYNVTQAFGRRGPEQTQGNFGDQELIRQGTDYKHWPQIAQFAPSASAFFGMSRI
GMEVTPSGTWLTYTAAIKLDDKDPNFKDQVILLNKHIDAYKTFPPTEPKKDKKK
KADETQALPQRQKKQQTVTLLPAADLDDFSKQLQQSMSSADSTQA (SEQ ID NO:
1);
 and an oligolysine sequence having an amino acid sequence of RRRRR, and an linker peptide having an amino acid sequence of GSGS. An oligolysine sequence, an N protein and an oligolysine sequence were linked via the linker peptide, fusing into the recombinant N protein, which is encoded by the following DNA sequence:

ATGCGTCGCAGACGTCGAGGATCTGGCTCGATGAGTGATAACGGCCC
GCAGAATCAGCGCAATGCCCCGCGCATTACCTTTGGTGGCCCGAGTGATAGTA
CCGGTAGTAATCAGAATGGTGAACGCAGCGGCGCCCGCAGTAAACAGCGTCG
TCCGCAGGGTCTGCCGAATAATACCGCAAGCTGGTTTACCGCCCTGACCCAGC
ATGGCAAAGAAGATCTGAAATTTCCGCGCGGTCAGGGTGTTCCGATTAATACC
AATAGCAGTCCGGATGATCAGATTGGTTATTATCGTCGTGCCACCCGTCGTATT
CGCGGCGGCGATGGTAAAATGAAAGATCTGAGTCCGCGTTGGTATTTTTATTAT
CTGGGCACCGGTCCGGAAGCAGGCCTGCCTTATGGTGCCAATAAGGATGGCAT
TATTTGGGTGGCAACCGAAGGCGCCCTGAATACCCCGAAAGATCATATTGGTA
CCCGTAATCCGGCCAATAATGCAGCAATTGTGCTGCAGCTGCCGCAGGGCACC
ACCCTGCCTAAAGGCTTTTATGCAGAAGGTAGTCGCGGTGGCAGCCAGGCAA
GCAGCCGTAGTAGTAGCCGCAGTCGTAATAGCAGTCGCAATAGCACCCCGGGT
AGCAGTCGTGGTACCAGCCCGGCACGCATGGCAGGCAATGGTGGTGACGCCG
CACTGGCACTGCTGCTGCTGGATCGTCTGAATCAGCTGGAAAGCAAAATGAG
TGGCAAAGGTCAGCAGCAGCAGGGCCAGACCGTGACCAAAAAATCTGCCGC
AGAAGCCAGCAAAAAACCGCGTCAGAAACGTACCGCCACCAAAGCCTATAAT
GTGACCCAGGCATTTGGCCGTCGCGGCCCGGAACAGACCCAGGGTAATTTTG
GTGACCAGGAACTGATTCGCCAGGGCACCGATTATAAACATTGGCCGCAGATT
GCACAGTTTGCACCGAGTGCCAGTGCCTTTTTCGGCATGAGCCGCATTGGCAT
GGAAGTTACCCCGAGTGGTACCTGGCTGACCTATACCGGTGCCATTAAGCTGG
ATGATAAAGATCCGAATTTTAAAGATCAGGTTATTCTGCTGAACAAACATATTG
ATGCCTATAAAACCTTCCCGCCGACCGAACCGAAAAAAGATAAAAAGAAAAA
GGCAGATGAGACCCAGGCACTGCCGCAGCGCCAGAAAAAACAGCAGACCGT
GACACTGCTGCCGGCAGCAGATCTGGATGATTTTAGTAAACAGCTGCAGCAGA
GCATGAGTAGCGCCGATAGTACCCAGGCCGGATCTGGCTCGCGTCGCAGACGT
CGATAA (SEQ ID NO : 2)。

The DNA encoding the recombinant N protein was obtained by gene synthesis (General Biosystems (Anhui) Co., Ltd.). The DNA encoding the recombinant N protein had a NcoI site upstream and a HindIII site downstream. The DNA was digested with the corresponding restriction endonucleases and ligated into an expression vector pET-28a(+) digested with NcoI and HindIII to obtain a recombinant plasmid pET-28a(+)-N.

The recombinant plasmid was transformed into *E. coli* expression strain BL21(DE3), and a single clone was picked, inoculated into 100 mL of LB medium containing 50 µg/mL kanamycin, and cultured overnight at 200 rpm at 37°C. The next day, 1% volume of the overnight culture was pipetted into 1 L of fresh LB medium containing 50 µg/mL kanamycin, and cultured at 200 rpm at 37°C until OD₆₀₀ reached about 0.6, then induced with IPTG (isopropyl thiogalactoside) at a final concentration of 1 mM for 20 h at 18 °C for expression. The bacteria cells were collected by centrifugation at 12,000 g for 3 min at 4°C, and the bacteria cells in one liter of bacterial solution were suspended with 40 mL buffer (Buffer A, 50 mM Na₂HPO₄, pH 7.4) pre-cooled on ice. After adding a small amount of RNase, the mixture was crushed by a high-pressure homogenizer, centrifuged at 12,000 g for 30 min at 4°C. The supernatant was filtered through a 0.22 µm filter membrane and passed through a cation exchange column.

The cation exchange column was equilibrated with 10 column volumes of buffer A, then the supernatant was added, and unbound protein was washed away with at least 10 column volumes of buffer A. A buffer (Buffer B, 50 mM Na₂HPO₄/500 mM NaCl, pH 7.4) was prepared, and the target protein was eluted with 20 column volumes of a linear gradient of the buffer containing 0 mM to 500 mM NaCl. The target protein fractions with a purity of 90% were selected and pooled, and fully dialyzed in a buffer (Buffer C, 10 mM Tris 7.5) at 4°C. In order to further remove impurities, the dialyzed N protein was loaded onto an anion exchange column pre-equilibrated with Buffer C, and the breakthrough fluid was collected and fully dialyzed in a PBS buffer. The N protein was concentrated by ultrafiltration before stored at -20°C for later use.

The purity of the target protein obtained through the above purification steps may reach 95% or more. SDS-PAGE shows no undesired bands can be seen in lanes loaded with various loading amounts (from 2 µg to 10 µg). The results are shown in FIG. 1.

The respective technical features of the above-mentioned embodiments can be combined arbitrarily. For the sake of brevity, not all the possible combinations of each of the technical features in the above embodiments are described. However, all of the combinations of these technical features should be considered as within the scope of this disclosure, as long as such combinations do not contradict with each other.

The above-mentioned embodiments are merely illustrative of several embodiments of the present disclosure, which are described specifically and in detail, but should not to be construed as limiting the scope of the present disclosure. It should be noted that, for those ordinary skilled in the art, several variations and improvements may be made without departing from the concept of the present disclosure, and all of which are within the protection scope of the present disclosure. Therefore, the protection scope of the present disclosure shall be defined by the appended claims.

## Claims

1. A recombinant N protein of SARS-CoV-2 having an N protein sequence as set forth in SEQ ID NO: 1, wherein each of both ends of the N protein sequence are respectively linked to an oligolysine fragment.

2. The recombinant N protein according to claim 1, wherein the oligolysine fragment at the both ends of the N protein sequence independently comprises 4 to 6 lysines;
optionally, the N protein sequence is linked to the oligolysine fragment via a linker peptide;
optionally, the linker peptide is a flexible linker peptide;
optionally, the linker peptide has an amino acid sequence having one or more amino acids selected from the group consisting of Gly, Ser, Pro, Ala and Glu; and
optionally, the sequence of the linker peptide is GSGS.

3. A nucleic acid capable of expressing the recombinant N protein of claim 1 or 2, optionally, the nucleic acid has a sequence as set forth in SEQ ID NO:2.

4. A vector comprising the nucleic acid of claim 3.

5. A host cell incorporating the nucleic acid of claim 3 in genome thereof.

6. A method for preparing an N protein of SARS-CoV-2 comprising:
culturing the host cell of claim 5 under a suitable condition, and collecting a lysate of the host cell and/or culture solution.

7. A method for purifying an N protein of SARS-CoV-2 comprising:
step a) providing a sample comprising the recombinant N protein of claim 1 or 2; and
step b) performing cation exchange chromatography resin purification treatment at least once, and collecting breakthrough substance,
wherein, optionally, the cation exchange chromatography resin is SP Sepharose FF,
optionally, the breakthrough substance in step b) is a target protein fraction with a purity of 90%, and step b) further comprises subjecting the breakthrough substance to dialysis treatment at least once.

8. The method according to claim 7, wherein the method further comprises, after step b), performing at least one anion exchange chromatography resin purification treatment on the obtained product at least once, and collecting breakthrough substance.

9. A composition obtained by purification by the method of claim 7 or 8.

10. A reagent or kit for detecting an antibody against N protein of SARS-CoV-2, comprising the recombinant N protein of claim 1 or 2, or the composition of claim 9.
